(19) Europäisches Patentamt
European Patent Office
Office européen des brevets

(11)  EP 4 349 855 A1

(12)  **EUROPEAN PATENT APPLICATION**
published in accordance with Art. 153(4) EPC

(43) Date of publication:
10.04.2024  Bulletin 2024/15

(21) Application number: 22809977.6

(22) Date of filing: 03.05.2022

(51) International Patent Classification (IPC):
*C07K 14/44* $^{(2006.01)}$   *A61K 39/005* $^{(2006.01)}$
*A61P 33/02* $^{(2006.01)}$

(52) Cooperative Patent Classification (CPC):
**A61K 39/005; A61P 33/02; C07K 14/44;**
Y02A 50/30

(86) International application number:
**PCT/BR2022/050150**

(87) International publication number:
**WO 2022/246526 (01.12.2022 Gazette 2022/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  28.05.2021  BR 102021010469

(71) Applicant: **Fundação Oswaldo Cruz**
**21045-900 Rio de Janeiro, RJ (BR)**

(72) Inventors:
• **GAZZINELLI, Ricardo, Tostes**
**30112-000 Belo Horizonte, MG (BR)**
• **CASTRO, Júlia, Teixeira, de**
**30170-040 Belo Horizonte, MG (BR)**

• **JUNQUEIRA, Caroline, Furtado**
**30170-135 Belo Horizonte, MG (BR)**
• **TEIXEIRA, Santuza, Maria, Ribeiro**
**31365-570 Belo Horizonte, MG (BR)**
• **FERNANDES, Ana, Paula, Salles, Moura**
**30441-045 Belo Horizonte, MG (BR)**

(74) Representative: **Meissner Bolte Partnerschaft
mbB**
**Patentanwälte Rechtsanwälte
Postfach 10 26 05
86016 Augsburg (DE)**

Remarks:
The complete document including Reference
Table(s) and the Sequence Listing(s) can be
downloaded from the EPO website

(54)  **RECOMBINANT CHIMERIC PROTEIN, USE THEREOF, AND COMPOSITION**

(57)  The present invention relates to a recombinant chimeric protein containing immunogenic regions from the trans-sialidase (TS) protein and amastigote surface protein-2 (ASP-2) from *Trypanosoma cruzi* and a composition containing said protein that displayed vaccine potential in a murine model. The invention also comprises the use of the chimeric protein for manufacturing vaccines.

Figure 1

**Description**

The present invention relates to a recombinant chimeric protein comprising immunogenic regions of the trans-sialidase (TS) protein and of the *Trypanosoma cruzi* amastigote surface protein 2 (ASP-2) and a composition containing such protein which showed vaccine potential in a murine model. The invention further comprises the use of a chimeric protein for the manufacture of vaccines.

Chagas disease is caused by the intracellular protozoa *Trypanosoma cruzi,* belonging to the Trypanosomatidae family and which is currently a serious public health problem. Chagas disease is a neglected disease that mainly affects low-income populations, and, despite existing prophylaxis, the disease is still responsible for approximately 10 thousand deaths per year, with approximately 6 to 7 million people infected worldwide, especially in Latin America. Transmission occurs mainly by triatomine insect vectors belonging to the Triatoma, Panstrongylus and Rhodnius genera, although it can also occur through transfusion of infected blood, oral transmission through contaminated food, congenital transmission, organ transplantation or laboratory accidents.

The complex biological cycle of the parasite involves replication and extracellular differentiation of the parasite in the insect vector (triatomine) and intracellular differentiation in the vertebrate host. This favors its escape from immunological surveillance and persistence in the infected organism.

The existing medication for the treatment of Chagas disease in Brazil is benznidazole, which acts on both the trypomastigote and amastigote forms and is more effective in the acute phase of the disease. A study showed that the efficacy of benznidazole in the acute phase is 70 - 80 % (assessed by negative serology), but in the chronic phase it is only 8 %. A recent prospective study showed that the treatment of patients with established Chagas disease cardiomyopathy with benznidazole significatively reduced the detection of circulating parasites, but not disease progression. Therefore, the use of this drug in the chronic phase is still debatable. Furthermore, treatment can last up to 90 days and there are many adverse reactions, with allergic dermatitis occurring in around 30 % of patients, nausea, vomiting, anorexia, weight loss, insomnia and loss of appetite. A major problem with existing treatments is that they are more effective in the acute phase, when symptoms are non-specific, which is one of the major causes of mortality caused by Chagas disease.

Additionally, despite the implementation of systems for controlling and the preventing Chagas disease, the challenges for its eradication are still big.

Due to this fact, the development of a vaccine is needed. However, despite important advances in knowledge about Chagas disease since its characterization more than a century ago, the complexity of the host-parasite interaction has not yet allowed the production of an effective vaccine. Several initiatives have investigated the possibility of prophylactic and therapeutic vaccines against Chagas disease.

Some prior art documents describe vaccine compositions containing different antigens and several vaccination protocols.

Two proteins expressed by *T. cruzi* have been studied as potential vaccine antigens: Trans-sialidase (TS) and Amastigote surface protein 2 (ASP-2). TS is present mainly in the membrane of trypomastigotes, the extracellular stage of the parasite, and has a repetitive region in its C-terminal portion (SAPA) which induces an intense antibody production by B lymphocytes (SCHENKMAN et al., 1992; AFFRANCHINO et al., 1989). ASP-2 is typically found in the membrane of amastigotes, intracellular form of *T. cruzi.* In murine model, the immunization with plasmids containing the ASP-2 coding sequence is capable of inducing a potent cellular immune response, leading to the production of IFN-$\gamma$ by CD4+ and CD8+ T cells (VASCONCELOS et al., 2004).

The most effective vaccine protocol to date, tested on mice, uses an initial dose of plasmids containing the TS and ASP-2 genes and a booster dose of adenovirus type 5 (Ad5) which also encodes these proteins (PIAd-TS/ASP). It has been shown that this immunization is capable of protecting not only resistant mice (C57BL/6), but also the most susceptible (A/Sn) to infection with the Y strain of *T. cruzi.* In addition, the vaccine also obtained satisfactory results in chronic experimental models, in which the animals were challenged with the Brazil and Colombiana myotropic strains (VASCONCELOS et al., 2004; ALENCAR et al., 2009; ARAÚJO et al., 2014).

Despite being potentially immunogenic and strongly inducing a cellular response, the use of DNA vaccines (with plasmid and / or viral vectors) is still under debate. The US Food and Drug Administration (FDA) sets out in its guide "Points to Consider on Plasmid DNA Vaccines for Preventive Infectious Disease Indications" (1996, updated in 2007) some contingents that should be considered by vaccine developers. It was described the possibility of generating autoantibodies, which could lead to an autoimmune disease; the induction of immunological tolerance rather than immunogenicity; and also, the risk of plasmid DNA being integrated into the genome of host cells, increasing the chance of carcinogenesis. (KLINMAN et al., 1997; MOR et al., 1997; KLINMAN et al., 2000).

As regards viral vectors, there is concern about the possibility of virulence reversal, mainly through gene recombination between the wild virus and its attenuated version (CONDIT et al., 2016). Another factor to consider is the preexistence of immunity to the vector, which nullifies the vaccine effect, as reported in the clinical trial of an HIV vaccine using Ad5 ("STEP" trial, 2005). In this study, it was observed that individuals with high antibody titers against Ad5 had a higher incidence of HIV infection when compared to the placebo group (GRAY et al., 2010).

Patent document US9028844, entitled "Vaccine against Trypanosoma cruzi infection" whose priority date is March 17, 2006, describes a vaccine against *Trypanosoma cruzi* infection, useful in the treatment and / or prevention of Chagas disease. The vaccine of the aforementioned invention comprises a mutant and recombinant trans-sialidase.

Patent document US9250239, entitled "Compositions and methods for detecting microbial infections", whose priority date is February 07, 2014, describes a DNA vaccine for Chagas disease prophylaxis in which the DNA encoding the ASP-2 protein is used as a part of the glycoprotein-encoding plasmid described in the document.

Patent document AR100707, entitled "Polipéptido quimérico, vacunas contra la enfermedad de Chagas y método de inmunización" (in English, Chimeric polypeptide, vaccines against Chagas disease and immunization method), whose priority date is June 02, 2015, describes a polymeric peptide obtained from the sequences of a catalytic domain of cruzipain from *T. cruzi* and a region of the surface antigen Amastigote Surface Protein 2 (ASP2) from *T. cruzi,* joined by a connector that can be, in a preferred embodiment of the invention, a helix of the *T. cruzi* trans-sialidase.

The present invention deals with a chimeric protein whose sequence derives from the prediction of epitopes made with TS and ASP-2, using bioinformatics tools, in order to recognize the immunogenic regions, also considering the variability of HLAs in the population. By combining the immunogenic regions of both proteins, a chimeric protein was developed that showed vaccine potential in a murine model.

**BRIEF DESCRIPTION OF THE FIGURES**

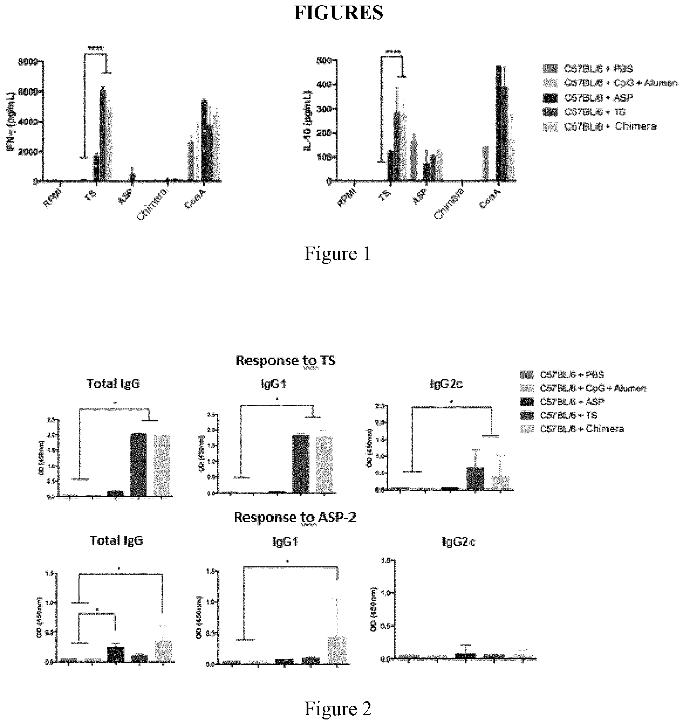

Figure 1 is a graphical representation of the Th1 cytokine response against TS and ASP-2 in immunized mice. The following groups were tested: PBS, administered with PBS buffer; CpG + Alumen, administered with CpG and aluminum hydroxide; and the ASP, TS and Chimera groups, immunized with trans-sialidase, amastigote surface protein 2 and the chimeric protein of the present invention, respectively, in individual doses of 100 $\mu$g/mL of protein + 180 $\mu$g/mL of CpG + 30 % v/v of aluminum hydroxide, in a total of 100 $\mu$L per mouse. Thirty days after the last booster, IFN-$\gamma$ and IL-10 production in spleen cells was assessed by ELISA of the culture supernatant. For this purpose, splenocytes from individual mice were stimulated for 48 hours with RPMI, Concanavalin A, TS, ASP-2 or Chimera. The optical density (OD) was measured at 450 nm. (4 mice / group). **** $p < 0.0001$.

Figure 2 is a graphical representation of the humoral immune response against TS and ASP-2 in immunized mice. The following groups were tested: PBS, administered with PBS buffer; CpG + Alumen, administered with CpG and aluminum hydroxide; and the ASP, TS and Chimera groups, administered with trans-sialidase, amastigote surface protein 2 and the chimeric protein of the present invention, respectively, in individual doses of 100 $\mu$g/mL of protein + 180 $\mu$g/mL of CpG + 30 % v/v of aluminum hydroxide, in a total of 100 $\mu$L per mouse. Twenty-one days after the last booster, the animals were bled and the presence of antibodies in the plasma was measured by ELISA. Anti-TS and anti-ASP2 total IgG, IgG1 and IgG2c were detected in diluted plasma at 1:800. The optical density (OD) was measured at 450 nm. (4 mice / group). * $p < 0.05$.

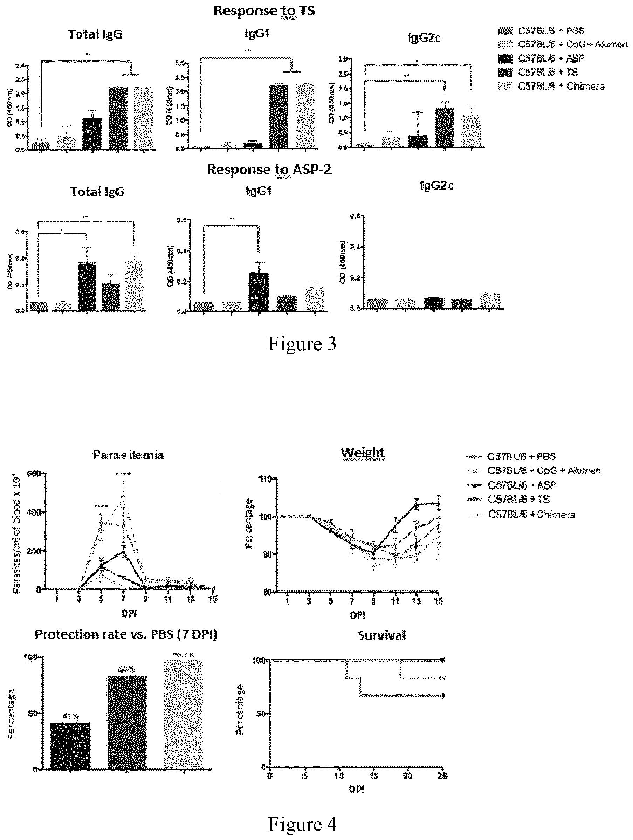

Figure 3 is a graphical representation of the humoral immune response against trans-sialidase (TS) and amastigote surface protein 2 (ASP-2) in immunized and challenged mice. The following groups were tested: PBS, administered with PBS buffer; CpG + Alumen, administered with CpG and aluminum hydroxide; and the ASP, TS and Chimera groups, immunized with trans-sialidase, amastigote surface protein 2 and the chimeric protein of the present invention, respectively, in individual doses of 100 $\mu$g/mL of protein + 180 $\mu$g/mL de CpG + 30 % v/v of aluminum hydroxide, in a total of 100 $\mu$L per mouse. Thirty days after the challenge, the animals were bled and the presence of antibodies in plasma was measured by ELISA. Anti-TS and anti-ASP2 total IgG, IgG1 and IgG2c were detected in diluted plasma at 1:800. The optical density (OD) was measured at 450 nm. * $p < 0,005$; ** $p < 0.001$.

Figure 4 is a graphical representation of the effects of the challenge with *T. cruzi* in immunized mice. The following groups were tested: PBS, administered with PBS buffer; CpG + Alumen, administered with CpG and aluminum hydroxide; and the ASP, TS and Chimera groups, immunized with trans-sialidase, amastigote surface protein 2 and the chimeric protein of the present invention, respectively, in individual doses of 100 $\mu$g/mL of protein + 180 $\mu$g/mL de CpG + 30 % v/v de aluminum hydroxide, in a total of 100 $\mu$L per mouse. The animals were infected with $10^4$ trypomastigotes of the Y strain and the course of infection by *T. cruzi* was measured by parasitemia. The protection rate was calculated using the mean parasitemia values of the vaccinated groups at 7 days post infection (DPI). The data is shown as a percentage and the mean value of the PBS group was considered to be 100 %. Weight was followed for 15 days after infection. Survival of all groups was followed for 36 days and is shown as Kaplan-Meier curves. (6 mice / group). **** $p < 0.0001$.

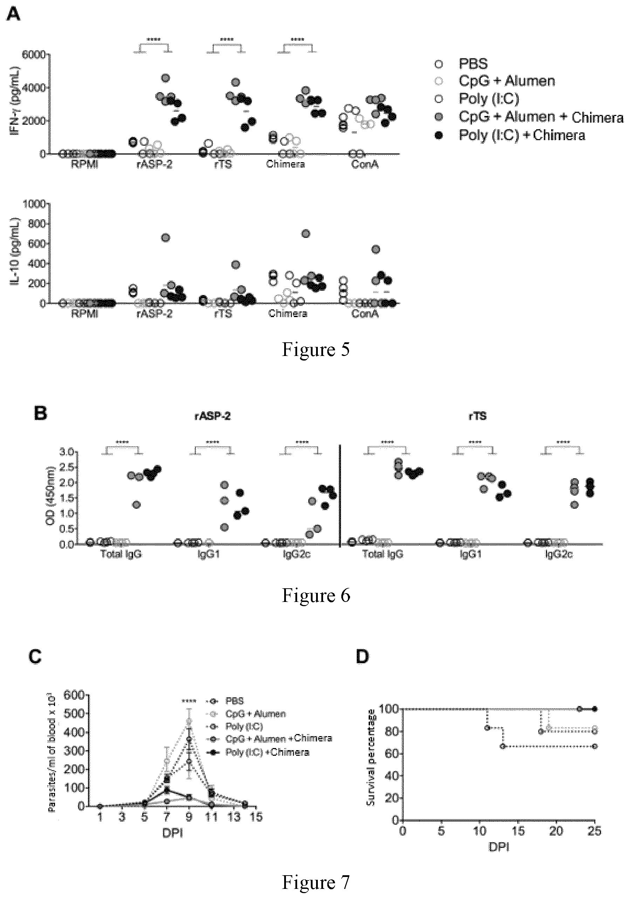

Figure 5 is a graphical representation of the detection of the cytokines IFN-$\gamma$ and IL-10 in splenocyte cultures from immunized mice with the chimeric protein included in two different vaccine formulations. The following groups were tested: PBS, administered with PBS buffer; CpG + Alumen, administered with CpG and aluminum hydroxide; Poly (I:C), administered only with Poly (I:C); CpG + Alumen + Chimera, which received 100 $\mu$g/mL of Chimera + 180 $\mu$g/mL of CpG + 30 % v/v of aluminum hydroxide; Poly (I:C) + Chimera, which received 100 $\mu$g/mL of Chimera + 500 $\mu$g/mL of Poly (I:C), in a final volume of 100 $\mu$l per mouse. Thirty days after the last booster, the production of IFN-$\gamma$ and IL-10 in spleen cells was assessed by ELISA of the culture supernatant. For this purpose, splenocytes from individual mice were stimulated for 48 hours with RPMI, Concanaval in A, TS, ASP-2 or Chimera. The optical density (OD) was measured at 450 nm. (4 mice / group). **** p < 0.0001.

Figure 6 is a graphical representation of the humoral immune response against trans-sialidase (TS) and amastigote surface protein 2 (ASP-2) in mice immunized with the chimeric protein included in two different vaccine formulations and challenged. The following groups were tested: PBS, administered with PBS buffer; CpG + Alumen, administered with CpG and aluminum hydroxide; Poly (I:C), administered only with Poly (I:C); CpG + Alumen + Chimera, which received 100 $\mu$g/mL of Chimera + 180 $\mu$g/mL of CpG + 30 % v/v of aluminum hydroxide; Poly (I:C) + Chimera, which received 100 $\mu$g/mL of Chimera + 500 $\mu$g/mL of Poly (I:C), in a final volume of 100 $\mu$l per mouse. Thirty days after the challenge, the animals were bled and the presence of antibodies in the plasma was measured by ELISA. Anti-TS and anti-ASP2 total IgG, IgG1 and IgG2c were detected in diluted plasma at 1:800. The optical density (OD) was measured at 450 nm. **** p < 0.0001.

Figure 7 is a graphical representation of the effects of challenge with *T. cruzi* in mice immunized with the chimeric protein included in two different vaccine formulations. The following groups were tested: PBS, administered with PBS buffer; CpG + Alumen, administered with CpG and aluminum hydroxide; Poly (I:C), administered only with Poly (I:C); CpG + Alumen + Chimera, which received 100 $\mu$g/mL of Chimera + 180 $\mu$g/mL of CpG + 30 % v/v of aluminum hydroxide; Poly (I:C) + Chimera, which received 100 $\mu$g/mL of Chimera + 500 $\mu$g/mL of Poly (I:C) , in a final volume of 100 $\mu$l per mouse. The animals were infected with $10^4$ trypomastigotes of the Y strain and the course of infection by *T. cruzi* was measured by parasitemia. The survival of all groups was monitored for 25 days and is shown as Kaplan-Meier curves (6 mice / group). **** p < 0.0001.

## SUMMARY OF THE INVENTION

In a first aspect, the present invention relates to a chimeric protein comprising a peptide sequence with at least 80 % identity with the SEQ ID N °1.

In an embodiment, the chimeric protein comprises the peptide sequence of SEQ ID N °1.

In a second aspect, the present invention relates to the use of a chimeric protein, according to the first aspect of the invention, for the manufacture of a composition for the prevention and / or treatment of infection by *Trypanosoma cruzi.*

In an embodiment, the composition is a vaccine.

In a second embodiment, the infection by *Trypanosoma cruzi* is Chagas disease.

In a third aspect, the invention refers to a composition comprising a chimeric protein, according to the first aspect of the invention, and adjuvants.

In an embodiment, the composition is a vaccine.

In a second embodiment, the adjuvants are selected from the group comprising CpG, aluminum hydroxide; monophosphoryl lipid A (MPL); polyinosinic-polycytidylic acid; and compositions containing mineral oils, vegetable oils, animal oils, compounds derived therefrom and / or combinations thereof. Preferably, the adjuvants are selected from the group comprising CpG, aluminum hydroxide; monophosphoryl lipid A (MPL); polyinosinic-polycytidylic acid; and compositions containing an emulsion of mineral oils, peanut oil, olive oil, sesame oil, soybean oil, wheat germ oil, grape seed oil, sunflower oil, castor oil, linseed oil, soybean oil, corn oil, copra oil, palm oil, walnut oil, hazelnut oil, rapeseed oil, squalane or squalene from olives or extracted from fish livers, or a combination thereof.

In another embodiment, the composition comprises 1 to 500 $\mu$g of protein, 10 to 500 $\mu$g/mL of CpG and 20 a 40 % v/v of aluminum hydroxide.

In another embodiment, the composition comprises 1 to 500 $\mu$g of protein, and 30 to 70 $\mu$g/mL of polyinosinic-polycytidylic acid.

## DETAILED DESCRIPTION OF THE INVENTION

As previously mentioned, the present invention relates to a protein for the prevention or treatment of *Trypanosoma cruzi* infections. Specifically, the present invention deals with a recombinant chimeric protein containing immunogenic regions of TS and ASP-2.

In a preferred embodiment, the recombinant chimeric protein of the present invention comprises a peptide sequence with at least 80 % identity with SEQ ID N° 1. In a more preferred embodiment, the protein comprises the peptide sequence SEQ ID N° 1.

The chimeric protein according to the present invention can be combined with any suitable vehicle or excipient, as known by a person skilled in the art, in order to increase its stability or provide a release at a specific site, an immediate or prolonged release, so as to provide a suitable composition. Preferably, according to the present invention, the composition is a vaccine composition.

According to a preferred embodiment, the composition of the invention comprises at least one immunologically active element against *Trypanosoma cruzi* and a pharmaceutically acceptable excipient. This element is preferably a recombinant chimeric protein comprising a peptide sequence with at least 80 % identity with SEQ ID N° 1. Even more preferably, the protein comprises the peptide sequence of SEQ ID N° 1.

For the preparation of the compositions according to the present invention, any methods known to those skilled in the art may be used.

As used herein, the term "immunologically active", or any reference to the immunological activity of an element, such as the protein of the invention, refers to the element's ability to stop, prevent or treat an infection by *Trypanosoma cruzi.*

As used herein, the term "biologically acceptable" refers to a vehicle or carrier that can be safely administered to an animal, particularly mammals and humans, without excessively negative or toxic side effects.

According to one embodiment of the invention, the composition can be formulated in the form of a pharmaceutical composition.

The present invention also relates to the use of a chimeric protein comprising a peptide sequence with at least 80 % identity with SEQ ID N° 1, for the manufacture of a composition for the prevention and / or treatment of *Trypanosoma cruzi* infection. Preferably, the protein comprises the peptide sequence of SEQ ID N° 1.

Although the compositions of the present invention are preferably administered parenterally, they can also be administered by any other suitable route. In a preferred embodiment, the pharmaceutical compositions may be administered intravenously, intramuscularly, or subcutaneously.

Examples of a preparation suitable for parenteral administration include injection, intravenous fluid, solution or suspension for infusion, lyophilized powder for suspension or solution, inhalation powder, and the like.

The formulation according to the present invention may also be in a form for oral administration. Examples of a preparation suitable for oral administration include tablet, capsule, powder, fine granule, pellet, solution, suspension, syrup, and the like.

However, the form of the preparation should not be limited to these alone.

As a pharmaceutically acceptable excipient, for example, an adjuvant, disintegrant or disintegrant auxiliary, binder, coating agent, colorant, diluent, base, solubilizer or solubilizer auxiliary, isotonicity agent, pH regulator, stabilizer, propellant, adhesive and the like can be used.

A preparation suitable for oral administration may contain, as an excipient, for example, glucose, lactose, lactose monohydrate, D-mannitol, starch, crystalline cellulose and the like; disintegrant or disintegrant auxiliary, such as carboxymethylcellulose, starch, calcium carboxymethylcellulose, silicon dioxide and the like; binder, such as hydroxypropylcellulose, hydroxypropylmethylcellulose, polyvinylpyrrolidone, gelatine and the like; lubricant, such as magnesium stearate, talc and the like; base, such as hydroxypropylmethylcellulose, sucrose, polyethylene glycol, gelatine, kaolin, glycerol, purified water, hard fat, and the like.

A preparation suitable for injection or intravenous fluid may contain pharmaceutical excipients for preparation, such as solubilizer or solubilizing auxiliary, capable of constituting an aqueous injection or an injection to be dissolved when in use, such as in distilled water for injection, saline solution, propylene glycol, and the like; isotonicity agent, such as glucose, sodium chloride, D-mannitol, glycerol, and the like; pH regulator such as an inorganic acid, organic acid, inorganic or organic base or the like.

According to the present invention, the adjuvants are preferably selected from the group comprising CpG and aluminum hydroxide.

In general, the appropriate dose can be administered in one to several portions or can be administered every few days according to a defined initial - boost dose protocol. In a preferred embodiment of the invention the composition comprises 1 to 500 $\mu$g of protein, 10 to 500 $\mu$g/mL of CpG, preferably 180 $\mu$g/mL, 20 to 40 % v/v of aluminum hydroxide, preferably 30 % v/v. In another preferred embodiment of the invention the composition comprises 1 to 500 $\mu$g of protein and 100 to 1000 $\mu$g/mL of Poly-IC, preferably 500 $\mu$g/mL.

According to the present invention, the disease caused by *Trypanosoma cruzi* infection is Chagas disease.

The present invention is further described by the following non-limiting examples, which are merely illustrative. Several modifications and variations of the embodiments are evident to the person skilled in the art, without departing from the spirit and scope of the invention.

Several variations to the scope of protection of the present application are allowed. This reinforces the fact that the present invention is not limited to the particular configurations / embodiments described above.

**Example 1- Prediction of TS and ASP-2 epitopes and modeling of recombinant chimeric protein**

Using the programs SYFPEITHI, Bimas and *The Immune Epitope Database and Analysis Resource* (IEDB), epitopes of the TS and ASP-2 proteins were predicted. From the epitopes found, the five best ranked for each HLA-ABC allele were selected, i.e., those with the highest probability of binding to MHC class I. Subsequently, the frequency with which the same peptide appeared among the top five was evaluated for all the HLA alleles analyzed, in search of immunodominant epitopes. The peptides were highlighted by colors in the amino acid sequence of the proteins, according to their frequency. After identifying these regions, the chimeric protein and sequence (SEQ ID N°1) was defined by joining the immunogenic region of the TS protein with that of the ASP-2 protein. In addition, three SAPA repeats were added, present in the TS protein, which, as described in previous studies, induce an intense antibody production. Therefore, the sequence of the chimeric protein includes the TS portion from amino acids 1 to 234, followed by SAPA (aa 235 to 270), a spacer region of three glycines (aa 271 to 273) and finally the ASP-2 region (aa 274 to 573).

**Example 2 - Vaccination with Chimera protein and challenge**

The trials were carried out on C57BL/6 mice (6 animals / group). Vaccination consists of an initial dose and two booster doses, all with the same constructs, with an interval of 21 days from one to the other. One group receives the TS protein, another ASP-2 (only the regions contained in Chimera) and another the Chimera protein (SEQ ID N° 1) in individual doses of 100 $\mu$g/mL of protein + 180 $\mu$g/mL of CpG + 30 % v/v of aluminum hydroxide, in a final volume of 100 $\mu$L per mouse. CpG and aluminum hydroxide are vaccine adjuvants. The injection is made subcutaneously in the dorsal region. One control group receives only the adjuvants and the other only PBS. Thirty days after the last booster, part of the animals is euthanized to analyze the immune response and part is challenged with $10^4$ trypomastigote forms of *T. cruzi* strain Y to evaluate parasitemia.

From the animals that were euthanized 30 days after the booster, in order to assess the efficacy of the vaccination, the spleen was removed to obtain splenocytes. The cells obtained were incubated with erythrocyte lysis solution (Ammonium-Chloride-Potassium - ACK), washed with RPMI 1640 medium containing 1 % of antibiotic and counted. The splenocytes were then plated in 96-well plates ($1 \times 10^6$ cells per well) and stimulated for 48 hours with the proteins TS, ASP-2 and Chimera, individually, at a concentration of 10 $\mu$g/mL. After the incubation period, the plates were centrifuged, and the culture supernatant was stored at -20 ºC. Subsequently, the levels of IFN-$\gamma$ and IL-10 in the culture supernatant were assessed using the enzyme-linked immunosorbent assay (ELISA) technique, following the manufacturer's instructions (Mouse IFN-$\gamma$ DuoSet ELISA, R&D Systems). The samples were assessed in duplicate and the colorimetric reaction was read in a microplate reader at 450 nm.

The results are shown in Figure 1. As expected, the animals that were not vaccinated (vaccine control and PBS) were unable to activate an immune response and produce cytokines. On the other hand, the animals vaccinated with TS and Chimera were able to secrete IFN-$\gamma$ and IL-10 upon stimulation with the TS protein.

To assess the specific humoral response before the challenge, blood samples (taken through the orbital plexus) from the immunized and challenged animals were collected 30 days after the last boost. The blood was centrifuged, and the plasma obtained was stored at -20 ºC. Subsequently, antibody production was assessed using the ELISA technique. Initially, a 96-well plate was sensitized with 5 $\mu$g of TS or ASP-2, and incubated overnight at 4 °C. Subsequently, the animals' plasma was added (in duplicate) at a dilution of 1:800. This was followed by incubation with secondary antibodies for total IgG, IgG1 and IgG2a conjugated to streptavidin-HRP (dilution 1:5,000). The colorimetric reaction was read at 450 nm. As shown in Figure 2, before the challenge, the animals vaccinated with TS and Chimera produced high rates of total IgG and anti-TS IgG1, mainly.

To evaluate the specific humoral response after challenge, blood samples (taken through the orbital plexus) from immunized and challenged animals were collected 30 days after challenge with *T. cruzi.* The blood was centrifuged, and the plasma obtained was stored at -20 ºC. Subsequently, the production of antibodies was assessed using the ELISA technique. Initially, a 96-well plate was sensitized with 5 $\mu$g of TS or ASP-2, and incubated overnight at 4 °C. Subsequently, the animals' plasma was added (in duplicate) at a dilution of 1:800. This was followed by incubation with secondary antibodies for total IgG, IgG1 and IgG2a conjugated to streptavidin-HRP (dilution 1:5,000). The colorimetric reaction was read at 450 nm. The result is shown in Figure 3.

Once again, the production of total IgG and IgG1 anti-TS was observed in animals vaccinated with TS and Chimera. In addition, in these groups there was also an increase in the production of anti-TS IgG2, and of total IgG and anti-ASP-2 IgG1.

**For parasitemia assessment**

Parasitemia was assessed 15 days post-challenge by viewing the parasites under a microscope. According to the Pizzi-Brener method (1962), 5 $\mu$L of blood collected from the animal's tail was placed between a slide and a coverslip (20 mm × 20 mm) and 50 random fields were evaluated. After counting, the total number of parasites per mL is calculated using the following formula:

$$\textit{Parasitemia = number of parasites counted x microscope correction factor x 200 (to arrive at a value of parasites in 1 mL)}$$

It was observed that the animals vaccinated with Chimera were more resistant to infection, showing parasitemia 96.7 % lower than the PBS group on the seventh day post-infection (DPI). On the other hand, the animals vaccinated with TS and ASP-2 had partial protection.
During the course of the infection, the animals' weight was measured. To make it easier to see the differences between the groups, the percentage change in weight was calculated, with the initial weight taken as 100%.
In addition, the deaths that occurred during the experiment were recorded. As shown in Figure 4, the three vaccinated groups showed 100 % survival.

**Example 3** - **Adjuvant comparison**

Subsequently, a comparison of vaccine formulations was carried out using Chimera supplemented with two different adjuvants. Because it showed excellent results in human clinical trials for cancer and HIV vaccines (AMMI *et al.*, 2015; SAXENA *et al.*, 2019), polyinosinic:polycytidylic acid (Poly (I:C)) was the adjuvant chosen to be compared with CpG and Alumen.
The formulation of Chimera + CpG + Alumen was maintained at the concentrations described above. For Chimera + Poly (I:C), 100 $\mu$g/mL of protein + 500 $\mu$g/mL of Poly (I:C) were used, in a final volume of 100 $\mu$L per dose. The vaccine protocol was the same as previously used, consisting of three vaccine doses with an interval of twenty-one days between administrations.
The cellular immune response was induced in different experimental groups by detecting the cytokines IFN-$\gamma$ and IL-10 in the splenocyte culture supernatant. In this experimental trial, in addition to the stimuli with rASP-2 and rTS, Chimera was added.
As can be seen in **Figure** 5, animals immunized with Chimera, regardless of the adjuvant used [CpG + Alumen or Poly (I:C)], were able to secrete IFN-$\gamma$ in the presence of rASP-2, rTS or Chimera. It is important to note that the three recombinant proteins were able to stimulate the splenocytes at the same intensity, with no difference between the stimuli. The immunized groups showed higher levels of IFN-$\gamma$ than the control groups. This difference was more pronounced between the PBS and rTS-stimulated groups (~ 25 times greater).
The production of IL-10 (**Figure** 5) occurred at a lower rate than that of IFN-$\gamma$. However, it was possible to observe statistically significant differences between the groups immunized with CpG + Alumen in relation to the control groups, both in the presence of the stimulus with rASP-2 and Chimera.
The humoral immune response was assessed by the detection of total IgG, IgG1 and IgG2c antibodies specific to the rASP-2 and rTS proteins. It can be seen that both immunization with CpG + Alumen + Chimera and with Poly (I:C) + Chimera stimulated the production of anti-rASP-2 (**Figure** 6) and anti-rTS (**Figure** 6) antibodies. Furthermore, it is important to note that the use of different adjuvants was not able to change the production of total IgG and IgG1 specific for rASP and rTS. However, it should be noted that Chimera associated with the Poly (I:C) adjuvant induced a twofold greater production of IgG2c (a typical subclass of Th1-type responses) against rASP-2, compared to the use of the CpG + Alumen adjuvant. As expected, none of the antibody types were produced in the control groups.
In order to compare the ability of the two vaccine formulations to induce an antiparasitic response, immunized mice were challenged with $10^4$ trypomastigotes of the Y strain from *T. cruzi*. Parasitemia was assessed every 2 days for 15 days after the challenge, starting on the third day after infection. As can be seen in **Figure** 7, the mice immunized with Chimera, regardless of the adjuvant used, showed the lowest rates of parasitemia when compared to the control groups. This shows, therefore, that the two formulations were effective in preventing experimental *T. cruzi* infection. In addition, both immunized groups had a 100 % survival rate (**Figure** 7). In contrast, the immunization control groups, CpG + Alumen and Poly (I:C), had a survival rate of 83.3 % and 80 % respectively. The experimental control group (PBS), on the other hand, had a survival rate of only 66.67 %

**Claims**

1. Chimeric protein **characterized by** comprising a peptide sequence with at least 80 % identity with the SEQ ID N° 1.

2. Chimeric protein **characterized by** comprising the peptide sequence of SEQ ID N° 1.

3. Use of a chimeric protein, as defined in claim 1 or 2,
   **characterized by** being for the manufacture of a composition for the prevention and / or treatment of an infection by *Trypanosoma cruzi.*

4. Use, according to claim 3,
   **characterized by** the composition being a vaccine.

5. Use, according to claims 3 or 4,
   **characterized by** the *Trypanosoma cruzi* infection being Chagas disease.

6. Composition **characterized by** comprising a chimeric protein, as defined in claim 1 or 2, and adjuvants.

7. Composition, according to claim 6,
   **characterized by** being a vaccine.

8. Composition, according to claim 6 or 7,
   **characterized by** the adjuvants being selected from the group comprising CpG, aluminum hydroxide; monophosphoryl lipid A (MPL); polyinosinic-polycytidylic acid; and compositions containing mineral oils, vegetable oils, animal oils, compounds derived therefrom and / or combinations thereof.

9. Composition, according to any one of claims 6 to 8,
   **characterized by** comprising:

   - 1 to 500 $\mu$g of said protein;
   - 10 to 500 $\mu$g/mL of CpG, preferably 180 $\mu$g/mL; and
   - 20 a 40 % v/v of aluminum hydroxide, preferably 30 % v/v.

10. Composition, according to any one of claims 6 to 8,
    **characterized by** comprising:

    - 1 to 500 $\mu$g of said protein; and
    - 100 to 1000 $\mu$g/mL of polyinosinic-polycytidylic acid, preferably 500 $\mu$g/mL.

## FIGURES

Figure 1

Figure 2

Figure 3

Figure 4

**A**

Figure 5

**B**

Figure 6

**C**

**D**

Figure 7

## INTERNATIONAL SEARCH REPORT

| International application No. |
|---|
| **PCT/BR2022/050150** |

**A.    CLASSIFICATION OF SUBJECT MATTER**

C07K 14/44 (2006.01)i; A61K 39/005 (2006.01)i; A61P 33/02 (2006.01)

According to International Patent Classification (IPC) or to both national classification and IPC

**B.    FIELDS SEARCHED**

Minimum documentation searched (classification system followed by classification symbols)

Nenhuma

Documentation searched other than minimum documentation to the extent that such documents are included in the fields searched

Base de patentes do INPI-BR, Plataforma Lattes (http://lattes.cnpq.br/).

Electronic data base consulted during the international search (name of data base and, where practicable, search terms used)

DWPI, GENESEQ, USGENE, WOGENE, GENBANK, XP3GPP, XPCPVO, XPIETF, XPIPCOM, XPJPEG, XPMISC, XPOAC, XPTK, MEDLINE, TDB, NPL, EPODOC.

**C.    DOCUMENTS CONSIDERED TO BE RELEVANT**

| Category* | Citation of document, with indication, where appropriate, of the relevant passages | Relevant to claim No. |
|---|---|---|
| X<br><br>A | US 8900598 B2 (DE BAEREMAECKER BARROS CARLOS  [UY]) 02 December 2014 (2014-12-02)<br>    Abstract, column 4 lines 53-56, column 5 lines 13-34, column 12 lines 1-8 and 61-67, SEQ ID NO 6. | 1, 3-10<br><br>2 |
| A | MACHADO, A. V. et al. Long-term protective immunity induced against Trypanosoma cruzi infection after vaccination with recombinant adenoviruses encoding amastigote surface protein-2 and trans-sialidase. Human Gene Therapy. 2006. Vol. 17, no. 9, pages 898-908. doi: 10.1089/hum.2006.17.898. Abstract, Materials and Methods, page 906, tables 1 and 2, figures 1, 4 and 5. | 1-10 |
| A | VASCONCELOS, J. R. et al. Protective Immunity Against Trypanosoma cruzi Infection in a Highly Susceptible Mouse Strain After Vaccination with Genes Encoding the Amastigote Surface Protein-2 and Trans-Sialidase. Human Gene Therapy. 2004. Vol. 15, no. 9, pages 878-886, doi: 10.1089/hum.2004.15.878. Abstract, page 885 second column. | 1-10 |

☑ Further documents are listed in the continuation of Box C.          ☑ See patent family annex.

| * Special categories of cited documents: | "T" later document published after the international filing date or priority date and not in conflict with the application but cited to understand the principle or theory underlying the invention |
|---|---|
| "A"   document defining the general state of the art which is not considered to be of particular relevance | |
| "D"   document cited by the applicant in the international application | "X"   document of particular relevance; the claimed invention cannot be considered novel or cannot be considered to involve an inventive step when the document is taken alone |
| "E"   earlier application or patent but published on or after the international filing date | |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | "&"   document member of the same patent family |
| "P"   document published prior to the international filing date but later than the priority date claimed | |

| Date of the actual completion of the international search | Date of mailing of the international search report |
|---|---|
| **15 June 2022** | **22 June 2022** |

| Name and mailing address of the ISA/BR | Authorized officer |
|---|---|
| **National Institute of Industrial Property (Brazil)**<br>**Rua Mayrink Veiga, 9, 6º andar, CEP 20.090-910 Rio de Janeiro – RJ**<br>**Brazil** | **Flávia Riso Rocha** |
| Telephone No. **(55 21) 3037-3742, 3037-3984** | Telephone No. **55 21 987823799** |

Form PCT/ISA/210 (second sheet) (July 2019)

## INTERNATIONAL SEARCH REPORT

International application No.

**PCT/BR2022/050150**

### C.    DOCUMENTS CONSIDERED TO BE RELEVANT

| Category* | Citation of document, with indication, where appropriate, of the relevant passages | Relevant to claim No. |
|---|---|---|
| A | DUMONTEIL, E. et al. The Case for the Development of a Chagas Disease Vaccine: Why? How? When? Trop Med Infect Dis. January 2021. Vol. 6, no. 1: 16. 14 pages. doi: 10.3390/tropicalmed6010016 Abstract, page 7 second paragraph, table 1. | 1-10 |
| A | PEREIRA, I. R. et al. A human type 5 adenovirus-based Trypanosoma cruzi therapeutic vaccine re-programs immune response and reverses chronic cardiomyopathy. PLoS Pathog. 2015. Vol. 11, no. 1:e0191596. doi: 10.1371/journal.ppat.1004594. Abstract, Discussion, Materials and Methods. | 1-10 |
| A | ARAÚJO, A. F. S. et al. CD8+-T-cell-dependent control of Trypanosoma cruzi infection in a highly susceptible mouse strain after immunization with recombinant proteins based on amastigote surface protein 2. Infect Immun. 2005. Vol. 73, no. 9, pages 6017-25. doi: 10.1128/IAI.73.9.6017-6025.2005. Abstract, Materials and Methods, page 6023 second column - page 6024 first column, figure 1, table 1. | 1-10 |
| A | ALBERTI, A. S. et al. Engineered trivalent immunogen adjuvanted with a STING agonist confers protection against Trypanosoma cruzi infection. npj Vaccines volume. 2017. Vol. 2, Article number: 9. doi: https://doi.org/10.1038/s41541-017-0010-z Abstract, Discussion, Materials and Methods. | 1-10 |
| A | CASTRO, J. T. et al. Immunization with Chimeric Protein induces protection against Trypanosoma cruzi infection in mice. In: XLIII Congress of the Brazilian Society of Immunology, 2018, Ouro Preto. XLIII Congress of the Brazilian Society of Immunology, 2018, page 61. The document as a whole. | 1-10 |
| A | BR PI0806285 A2 (UNIV MINAS GERAIS [BR]) 11 January 2011 (2011-01-11) Abstract, examples 1-5. | 1-10 |

Form PCT/ISA/210 (second sheet) (July 2019)

**INTERNATIONAL SEARCH REPORT**

| International application No. |
| --- |
| **PCT/BR2022/050150** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.   ☑ forming part of the international application as filed:

          ☑ in the form of an Annex C/ST.25 text file.

          ☐ on paper or in the form of an image file.

     b.   ☐ furnished together with the international application under PCT Rule 13*ter*.1(a) for the purposes of international search only in the form of an Annex C/ST.25 text file.

     c.   ☐ furnished subsequent to the international filing date for the purposes of international search only:

          ☐ in the form of an Annex C/ST.25 text file (Rule 13*ter*.1(a)).

          ☐ on paper or in the form of an image file (Rule 13*ter*.1(b) and Administrative Instructions, Section 713).

2.   ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
Information on patent family members

International application No.

**PCT/BR2022/050150**

| Patent document cited in search report | | | Publication date (day/month/year) | Patent family member(s) | | | Publication date (day/month/year) |
|---|---|---|---|---|---|---|---|
| BR | PI0806285 | A2 | 11 January 2011 | NONE | | | |
| US | 8900598 | B2 | 02 December 2014 | US | 2010297186 | A1 | 25 November 2010 |
| | | | | AR | 064593 | A1 | 15 April 2009 |
| | | | | BR | PI0805753 | A2 | 17 April 2012 |
| | | | | CA | 2702534 | A1 | 07 May 2009 |
| | | | | CA | 2702534 | C | 18 October 2016 |
| | | | | CO | 6280407 | A2 | 20 May 2011 |
| | | | | CR | 11334 | A | 26 May 2010 |
| | | | | DO | P2010000083 | A | 15 July 2010 |
| | | | | EC | SP10010055 | A | 29 June 2010 |
| | | | | EP | 2206513 | A2 | 14 July 2010 |
| | | | | EP | 2206513 | B1 | 17 February 2016 |
| | | | | ES | 2569943 | T3 | 13 May 2016 |
| | | | | GT | 201000066 | A | 03 April 2012 |
| | | | | HN | 2010000750 | A | 19 August 2013 |
| | | | | MX | 2010004626 | A | 04 October 2010 |
| | | | | NI | 201000071 | A | 10 November 2010 |
| | | | | PA | 8816401 | A1 | 17 September 2009 |
| | | | | PE | 20091161 | A1 | 28 August 2009 |
| | | | | PE | 20140520 | A1 | 05 May 2014 |
| | | | | US | 2015056243 | A1 | 26 February 2015 |
| | | | | US | 9662378 | B2 | 30 May 2017 |
| | | | | US | 2017290896 | A1 | 12 October 2017 |
| | | | | US | 10213500 | B2 | 26 February 2019 |
| | | | | UY | 31451 | A1 | 29 May 2009 |
| | | | | WO | 2009056965 | A2 | 07 May 2009 |
| | | | | WO | 2009056965 | A3 | 23 July 2009 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9028844 B
- US 9250239 B

- AR 100707